# EUROPÄISCHE PATENTSCHRIFT

(11) **EP 3 329 994 B1**
(45) Veröffentlichungstag und Bekanntmachung des Hinweises auf die Patenterteilung: **30.01.2019**
(21) Anmeldenummer: 17202689.0
(22) Anmeldetag: 21.11.2017
(51) Int. Cl.: B01L 3/00, B03B 5/00, B01D 15/12

(54) **REAKTOR ZUR ENZYMATISCHEN MAZERATION BIOGENER BESTANDTEILE EINER PARTIKELPROBE UND VERWENDUNG DES REAKTORS**
REACTOR FOR THE ENZYMATIC MACERATION OF BIOGENIC CONSTITUENTS OF A PARTICLE SAMPLE AND USE OF THE REACTOR
RÉACTEUR POUR LA MACÉRATION ENZYMATIQUE DES CONSTITUANTS BIOGÉNIQUES D'UN ÉCHANTILLON DE PARTICULES ET UTILISATION DU RÉACTEUR

(30) Priorität: 02.12.2016 DE 102016123324
(43) Veröffentlichungstag der Anmeldung: 06.06.2018
(73) Patentinhaber: Alfred-Wegener-Institut Helmholtz-Zentrum für Polar- und Meeresforschung, 27570 Bremerhaven (DE)
(72) Erfinder: Gerdts, Gunnar, 27498 Helgoland (DE)

(56) Entgegenhaltungen:
- EP-A2- 0 512 769
- Claudia Lorenz: "Detection of microplastics in marine sediments of the Germen Coanst via FT-IR spectrospcopy", , 22. Mai 2014 (2014-05-22), XP002780398, Gefunden im Internet: URL:http://rosdok.uni-rostock.de/file/rosd ok_thesis_0000000017/rosdok_derivate_00000 32921/Masterarbeit_Lorenz_2016.pdf [gefunden am 2018-04-20]
- MICHIEL CLAESSENS ET AL: "New techniques for the detection of microplastics in sediments and field collected organisms", MARINE POLLUTION BULLETIN, Bd. 70, Nr. 1-2, 1. Mai 2013 (2013-05-01), Seiten 227-233, XP055469528, GB ISSN: 0025-326X, DOI: 10.1016/j.marpolbul.2013.03.009
- HANNES K. IMHOF ET AL: "A novel, highly efficient method for the separation and quantification of plastic particles in sediments of aquatic environments : Novel plastic particle separation method", LIMNOLOGY AND OCEANOGRAPHY: METHODS, Bd. 10, Nr. 7, 1. Juli 2012 (2012-07-01), Seiten 524-537, XP55431254, US ISSN: 1541-5856, DOI: 10.4319/lom.2012.10.524

## Beschreibung

### Bezeichnung

Reaktor zur enzymatischen Mazeration biogener Bestandteile einer Partikelprobe und Verwendung des Reaktors

### Beschreibung

Die Erfindung bezieht sich auf einen Reaktor zur enzymatischen Mazeration biogener Bestandteile einer Partikelprobe mit zumindest einem Reaktionsraum, der als vertikaler Zylinder mit einer oberen Stirnseite und einer unteren Stirnseite ausgebildet ist, zumindest einer Filteranordnung, einem Pumpenanschluss und einem Flüssigkeitsanschluss und auf eine Verwendung des Reaktors.

Im Meer treibender Plastikmüll hat eine extrem lange Abbauzeit. Durch Witterung und Wasser zersetzt er sich aber langsam und wird zu Mikroplastik, das zum Gewässerboden sinkt und von Meerestieren aufgenommen wird. Die Verbreitung von Mikroplastik in der marinen Umwelt wird zunehmend dokumentiert. Als Mikroplastik werden Kunststoffpartikel mit einer Größe kleiner als 5 mm definiert. Eine untere Größenklasse kann nicht angegeben werden. In nahezu jeder Umweltprobe (Sediment, Wasser, Biota) sind mittlerweile Mikroplastikpartikel zu finden. Auch der Mensch kann hier betroffen sein, da die von den Kunststoffen abgegebenen Schadstoffe beispielsweise in Muscheln oder Seefischen akkumulieren und von ihm aufgenommen werden. Es bestehen daher verschiedene Ansprüche an die Forschung. Zum einen geht es um die Optimierung der Probennahme, des Nachweises, der Identität und der Quantifizierung der Mikroplastikpartikel. Zum anderen müssen grundlegende Studien zu ihrer Verteilung in der Umwelt und experimentelle Ansätze zu ihrer Wirkungsweise durchgeführt werden. Die vorliegende Erfindung befasst sich mit der Optimierung der Probenaufbereitung. Dabei geht es speziell darum, die die Mikroplastikpartikel enthaltenden Proben von allen weiteren partikulären, aber organischen Bestandteilen, beispielsweise Mikroalgen, Zooplankter, zu befreien und die Biofilme auf den Mikroplastikpartikeln selbst zu entfernen. Derartige Verunreinigungen würden ansonsten bei der Analytik mit den synthetischen Mikroplastikpartikeln interferieren und die Ergebnisse verfälschen. Verschiedene bekannte Lösungsansätze sehen entweder die Verwendung von aggressiven Säure-Laugen-Systemen oder von Enzymen vor. Allen bislang bekannten Lösungsansätzen ist eigen, dass diese meist in mehreren Schritten und mit händischen Eingriffen durchgeführt werden, sodass immer die Gefahr besteht, die Probe während des Aufbereitungsprozesses zusätzlich zu kontaminieren.

### Stand der Technik

Aus der österreichischen Patentschrift AT 231 923 B ist eine Vorrichtung zum Abspülen der anhaftenden Festbestandteile einer Suspension von Körnern einer feinkörnigen Gutes bekannt. Die Anordnung umfasst ein durchlässiges Transportband und ein darunter angeordnetes Sieb. Transportgut oberhalb des Siebes wird vertikal durchspült. Hierbei handelt es sich um eine Anordnung, die der Aufbereitung von Kohle oder Erzen dient. Aus der amerikanischen Patentschrift US 3 347 370 A ist ein Verfahren zum Waschen und Entfernen von organischen Schwerflüssigkeiten von mineralischen Partikeln bekannt. Es erfolgt eine Dichtetrennung. Aus der europäischen Patentschrift EP 0 316 622 B1 sind ein Verfahren und eine Anordnung zur Reinigung von polymerischen Harzteilchen bekannt. In einem zylindrischen Gehäuse verläuft vertikal der Partikelstrom. Horizontal wird auf der einen Gehäuseseite ein Hochdruckgasstrom angelegt, auf der anderen Seite wird ein Vakuum erzeugt. Stäube und kleine Partikel werden auf diese Weise aus dem Partikelstrom eliminiert.

Der der Erfindung nächstliegende Stand der Technik ist in dem Abschlussbericht "Mikroplastik in ausgewählten Kläranlagen des Oldenburgisch-Ostfriesischen Wasserverbandes (OOWV) in Niedersachsen" (von G. Gerdts et al., 08.10.2014, im Internet am 21.10.2016 abgerufen unter der URL http://www.dwa-bayern.de/tl_files/_media/content/PDFs/LV_Bayern/Abschlussbericht_Mikroplastik _in_Klaeranlagen-3.pdf) offenbart. In Kapitel 5 "Probenaufbereitung" werden Extraktionsschritte beschrieben, die erforderlich sind, um biogene und abiogene Bestandteile in den zu untersuchenden Proben voneinander zu trennen. Dazu wurden bei der Filtration von bis zu einem Kubikmeter geklärtem Abwasser neben Mikroplastik auch Pflanzenfragmente, Insekten, Sande und Fette auf Filter aus Edelstahl in zylindrischer Kerzenform ("Kerzenfilter") als Filteranordnung zurückgehalten. Bei der Entfernung des natürlichen Materials wurde bewusst auf den Einsatz von starken Säuren oder Laugen verzichtet, da solche auch das Potenzial haben Kunststoffe anzugreifen. Stattdessen wurden die Proben einer enzymatisch-oxidativen Behandlung ("enzymatische Mazeration") mit anschließender Dichteseparation mittels Zinkchlorid (ZnCl₂) unterzogen. Der Einsatz von spezifisch arbeitenden Enzymen hat den Vorteil, dass Pflanzenmaterial (Cellulase, Enzymaktivität > 30 U ml⁻¹), Fette (Lipase, Enzymaktivität > 15.000 U ml⁻¹), Proteine (Protease, Enzymaktivität 1.100 U ml⁻¹) und Insektenpanzer (Chitinase, Enzymaktivität > 40 U ml⁻¹) abgebaut, Kunststoffe aber nicht angegriffen werden. Die für den enzymatischen Abbau notwendige Sicherstellung der jeweiligen Optimalbedingungen hinsichtlich Temperatur und pH-Wert bedingte eine schrittweise und sehr zeitaufwändige Vorgehensweise. Nachdem mit Hilfe von gereinigter Druckluft die Reaktionsräume (Filtergehäuse) die Kerzenfilter entleert wurden, wurden mit Hilfe einer Vierfachmembranpumpe über einen Pumpenanschluss etwa 660 ml der einzelnen Chemikalien- bzw. Enzymlösungen über einen Flüssigkeitsanschluss direkt in die Reaktionsräume gepumpt. Auf diese Weise wurde zunächst eine fünfprozentige Lösung des Tensids SDS (Natriumdodecylsulfat) zugegeben. Anschließend wurde so auch mit den Enzymen Protease, Lipase und Cellulase verfahren, von welchen 25 ml pro Ablaufprobe zu zuvor angesetzten und pH- eingestellten Puffern gegeben wurden. Zwischen jedem Behandlungsschritt wurden die Kerzenfilter mit filtriertem Leitungswasser gespült und wieder entleert, um bereits gelöste Stoffe zu entfernen. Nach dem letzten Spülschritt wurden die Reaktionsräume geöffnet, das anhaftende Material mit einer Metallbürste von den Kerzenfiltern gelöst, mehrfach mit filtriertem Leitungswasser abgespült und über ein 500 µm Sieb auf einen 10 µm Scheibenfilter aus Edelstahl (Durchmesser 47 mm) filtriert. dabei besteht die Gefahr, dass die zu analysierenden Probenbestandteile nicht vollständig überführt werden. In der Fraktion größer 500 µm wurden alle potenziellen Plastikpartikel unter einem Binokular aussortiert und diese später mittels ATR-FTIR identifiziert. Die Probenfraktion kleiner 500 µm wurde auf den 10 µm Scheibenfiltern in Schottflaschen überführt und zunächst mit 30 ml H₂O₂ (35 %) versetzt. Nach dem Abfiltrieren wurde so auch mit Chitinase (2 ml in 30 ml Puffer) und erneuter H₂O₂-Behandlung verfahren. Für die finale Dichteseparation wurden die Proben von den Scheibenfiltern mit einer 1,6 g cm⁻³ dichten ZnCl₂-Lösung direkt in Scheidetrichter gespült. Alle zu erwartenden Kunststoffe hatten eine geringere Dichte, schwammen somit auf der Lösung, schwerere Stoffe wie Steine oder Rost sedimentierten und konnten so aus den Proben entfernt werden. Das verbliebene Material wurde anschließend direkt auf Scheibenfilter aus Aluminiumoxid filtriert. Die meisten Proben wiesen jedoch immer noch einen hohen Rückstand an organischem Material auf, wurden daher auf mehrere Scheibenfilter aufgeteilt, um gute Messergebnisse bei den anschließenden analytischen Untersuchungen erzielen zu können.

Eine ähnliche Offenbarung ist der Masterarbeit "Detection of microplastics in marine sediment of the German Coast via FT-IR spectroscopy" von C. Lorenz (Universität Rostock, 22.05.2014, abgerufen am 24.10. 2016 unter der URL http://rosdok.uni-rostock.de/file/rosdok_thesis_0000000017/rosdok_derivate_0000032921/Mastera rbeit_Lorenz_2016.pdf) zu entnehmen. Im Kapitel 2.3. "Separation" ist in der Figur 6 eine einen Reaktor darstellende Anordnung zu erkennen, mit deren Hilfe die Mazeration durchgeführt wird. Zu erkennen sind mehrere hintereinander geschaltete Kerzenfilter, wobei jeder Kerzenfilter eine Filteranordnung darstellt, in jeweils einem zylindrischen Reaktionsraum (Filtergehäuse) aus Glas. Die Kerzenfilter füllen jeweils den gesamten Reaktionsraum aus. Die Reaktionsräume weisen jeweils einen obere Stirnseite und eine untere Stirnseite auf. Der Pumpenanschluss ist gleichzeitig der Flüssigkeitsanschluss und befindet sich an der oberen Stirnseite des Reaktionsraumes. Eine optimale Durchströmung der Kerzenfilter ist damit nicht zwingend gewährleistet. Es handelt sich um einen weitgehend improvisierten Laboraufbau. Durch die separierten unterschiedlichen Verfahrensschritte und händischen Überführungsschritte (zwischen den Kerzenfiltern und den Scheibenfiltern, vergleiche oben) während der zeitaufwändigen Mazeration besteht die Gefahr der Probenkontamination. Außerdem kann es auftreten, dass die biogenen Stoffe in nicht ausreichendem Maße von den abiogenen Stoffen gelöst werden (vergleiche oben).

### Aufgabenstellung

Ausgehend von dem zuvor erläuterten nächstliegenden Stand der Technik ist die Aufgabe für die vorliegende Erfindung daher darin zu sehen, den gattungsgemäßen Reaktor so weiterzubilden, dass er eine möglichst hohe Mazerationseffizienz in Verbindung mit einer möglichst geringen Kontaminations- und Verlustgefahr der zu untersuchenden Partikel ermöglicht. Desweiteren soll der mit der Erfindung beanspruchte Reaktor Anwendung in einem vorteilhaften Verfahren zur Mazeration von Mikroplastikpartikel finden. Die Lösung für diese Aufgabe ist dem Hauptanspruch zu entnehmen. Vorteilhafte Weiterbildungen der Erfindung werden in den Unteransprüchen aufgezeigt und im Folgenden im Zusammenhang mit der Erfindung näher erläutert. Der Verfahrensanspruch zeigt eine Lösung für die Anwendungsaufgabe auf, zusammen mit möglichen Modifikationen wird das beanspruchte Anwendungsverfahren ebenfalls nachfolgend näher erläutert.

Der beanspruchte Reaktor ist erfindungsgemäß dadurch gekennzeichnet, dass an der oberen Stirnseite des Reaktionsraumes eine obere Filteranordnung mit einer oberen Filterronde zwischen zwei Dichtungen und an der unteren Stirnseite des Reaktionsraumes eine untere Filteranordnung mit einer unteren Filterronde zwischen zwei Dichtungen angeordnet ist, und dass auf der oberen Filteranordnung eine lösbare Kopfplatte mit dem Pumpenanschluss und auf der unteren Filteranordnung eine lösbare Bodenplatte mit dem Flüssigkeitsanschluss angeordnet ist, wobei Pumpenanschluss und Flüssigkeitsanschluss verschließbar und sowohl für eine Befüllung als auch für eine Entleerung des zylinderförmigen Reaktionsraumes ausgebildet sind.

Der erfindungsgemäße Reaktor stellt eine sehr kompakte und störungsunanfällige Anordnung zur Mazeration von Partikeln dar. Der Mazerationsvorgang mit unterschiedlichen Agenzien wird in nur einem Reaktionsraum durchgeführt, sodass Transferschritte zwischen unterschiedlichen Reaktionsräumen mit jeweils anderen Agenzien entfallen. Dabei können natürlich mehrere Reaktionsräume parallel angeordnet sein, sodass mehrere Mazerationsvorgänge zur Erhöhung des Aufbereitungsvolumens gleichzeitig durchgeführt werden können. Diese ist besonders vorteilhaft, um größere Probenmengen dem langwierigen Mazerationsprozess zu unterwerfen. Jeder zylindrische Reaktionsraum weist zwei Filteranordnungen auf. Jede Filteranordnung besteht aus einer Filterronde, die zwischen zwei Dichtungen eingespannt ist. Schwierig zu reinigende Kerzenfilter werden bei der Erfindung nicht verwendet.

Der Pumpenanschluss befindet sich im Bereich der oberen Filterronde, der Flüssigkeitsanschluss im Bereich der unteren Filterronde. Dadurch wird eine optimale Durchspülung des gesamten Reaktionsraumes beim Ein- und Ausfüllen der Flüssigkeit erreicht. Die untere Filterronde wird nach der Partikelablagerung auch für die anschließende analytische Untersuchung eingesetzt, sodass auch Transferschritte zwischen verschiedenen Filtertypen entfallen und Kontaminations- und Verlustrisiken minimiert werden. Die obere Filterronde dient dem Schutz der Pumpe vor Verstopfen durch Probenbestandteile, wenn die Pumpe im Absaugbetrieb läuft. Sowohl der Pumpenanschluss als auch der Flüssigkeitsanschluss sind verschließbar ausgebildet und dienen sowohl der Befüllung als auch der Entleerung des Reaktionsraumes. Zur Befüllung des Reaktionsraumes wird der Flüssigkeitsanschluss geöffnet und im Inneren des Reaktionsraumes ein Vakuum über den Pumpenanschluss erzeugt (Saugbetrieb der Pumpe, die am Pumpenanschluss hängt). Die Flüssigkeit wird über die untere Filterronde in das Innere des Reaktionsraumes gesogen. Der Flüssigkeitsanschluss wird anschließend verschlossen. Nach einem Mazerationsschritt wird die Flüssigkeit über die untere Filterronde aus dem Reaktionsraum ausgestoßen, in dem der Flüssigkeitsanschluss wieder geöffnet und über den Pumpenanschluss ein Überdruck im Reaktionsraum erzeugt wird (Förderbetrieb der Pumpe, die am Pumpenanschluss hängt). Die zu analysierenden und nunmehr durch Mazeration von biogenen Stoffen gereinigten abiogenen Partikel bleiben auf der Oberseite der unteren Filterronde zurück. Die so beladene Filterronde kann nunmehr unmittelbar der Analyse zugeführt werden. Durch die lösbare Kopfplatte (mit dem Pumpenanschluss) und die lösbare Bodenplatte (mit dem Flüssigkeitsanschluss) kann der Reaktionsraum einfach geöffnet werden. Die Sedimentprobe kann einfach in den Reaktionsraum eingebracht werden. Der Reaktionsraum kann nach Beendigung der Mazeration einfach gereinigt werden.

Bei dem analytischen Nachweis von Partikeln beispielsweise durch Infrarot-Spektroskopie, fallen Teilchen, die kleiner als 10 µm sind, unter die Nachweisgrenze. Sie tragen daher nicht zum Messergebnis bei und können aus der Sedimentprobe ausgespült werden. Damit sie die Filterronden passieren können, ist es gemäß einer ersten Ausführung der Erfindung deshalb bevorzugt und vorteilhaft, wenn die Filterronden als Edelstahlgaze mit einer Maschenweite von 10 µm ausgebildet sind. Der Edelstahl wird auch von dem Mazerationsprozess nicht angegriffen. weiterhin ist es bevorzugt und vorteilhaft, wenn die beiden Dichtungen ober- und unterhalb der Filterronden als Silikondichtringe ausgebildet sind. Diese dichten sehr gut, sind aber durch ihre guten Gleiteigenschaften auch wieder einfach lösbar. Dazu ist es vorteilhaft, wenn gemäß einer nächsten Erfindungsausgestaltung an den beiden Stirnseiten des zylinderförmigen Reaktionsraumes Flansche angeordnet sind (an der oberen Stirnseite ein oberer Flansch und an der unteren Stirnseite ein untere Flansch). dabei verlaufen die Flansche ringförmig um den Gehäuserand und behindern nicht den Zugang zur Mitte des Gehäuses. Weiterhin können bevorzugt und vorteilhaft sind die beiden Filteranordnungen mittels Spannklammern zwischen der Kopfplatte und dem oberen Flansch und der Bodenplatte und dem unteren Flansch lösbar eingespannt. Durch einfaches Lösen der Spannklammern können die Flansche entfernt und die Filterronden entnommen werden. Die untere Filterronde wird dann der Analyse zugeführt, die obere gereinigt.

Wesentlicher Teil der Mazeration ist die Inkubation. Die eingefüllten Agenzien reagieren mit den biogenen Stoffen und zersetzen sie. Dies erfolgt in warmer Umgebung, beispielsweise in einem Wasserbad, in bevorzugt liegender (horizontaler) Position des Reaktionsraumes. Dazu wird die Pumpe vom Pumpenanschluss getrennt. Dies kann einfach erfolgen, wenn gemäß einer nächsten bevorzugten und vorteilhaften Erfindungsausgestaltung der Pumpenanschluss mit einem druckdichten Ventil verschließbar ist. Weiterhin wird der Flüssigkeitsanschluss abgetrennt. Dazu ist es bevorzugt und vorteilhaft, wenn der Flüssigkeitsanschluss von einer einfachen Durchgangsbohrung in der Bodenplatte gebildet ist, wobei die Durchgangsbohrung mittels eines Kunststoffstopfens druckdicht verschließbar ist. Damit der Kunststoffstopfen beim Handhaben des Reaktionsraumes sicher in der Durchgangsbohrung verbleibt, ist es gemäß einer nächsten Ausgestaltung der Erfindung vorteilhaft und bevorzugt, wenn der Kunststoffstopfen mittels eines Gestellrahmens druckdicht in der Durchgangsbohrung halterbar ist. Der Gestellrahmen umfasst den Reaktionsraum im Bereich seiner unteren Stirnseite und kann gleichzeitig als Handhabungsgriff für den Reaktionsraum dienen. Zum dichten Einfügen, aber auch einfach Entnehmen des Stopfens aus dem ist es schließlich bevorzugt und vorteilhaft, wenn der Kunststoffstopfen aus Silikon besteht.

Der kompakte Reaktor nach der Erfindung eignet sich besonders für das langwierige Verfahren der stufenweisen Mazeration. Dazu wird die Sedimentprobe jeweils mit anderen Agentien in Kontakt gebracht, um jeweils andere biogene Stoffe von der Oberfläche der abiogenen Partikel zu entfernen. Ganz besonders bevorzugt und vorteilhaft ist die Verwendung des Reaktors in einer der zuvor erläuterten Ausführungsformen in einem Verfahren zur Trennung von biogenen und abiogenen Bestandteilen einer Probe durch enzymatische Mazeration aus einer Mikroplastikpartikel enthaltenden Planktonprobe (Anwendung als "Planktonreaktor") mit den Verfahrensschritten:
- Einfüllen der Planktonprobe durch die obere Stirnseite des zylinderförmigen Reaktionsraumes
- Abfiltern der Planktonprobe durch die untere Filterronde und die Durchgangsbohrung in der Bodenplatte mittels Schwerkraft
- Montieren der Kopfplatte mit dem Pumpenanschluss und dem druckdichten Ventil unter Zwischenlage der oberen Filteranordnung und der Dichtungen
- Platzieren des Reaktors in einem Gefäß, das mit einem Agens gefüllt ist,
- Befüllen des zylinderförmigen Reaktionsraumes mit dem Agens durch Erzeugen eines Vakuums am Pumpenanschluss
- Verschließen des Ventils im Pumpenanschluss und der Durchgangsbohrung in der Bodenplatte mit einem Kunststoffstopfen und Positionierung des Reaktors in einem Schüttelinkubator
- Inkubieren des Reaktors bei einer vorgegebenen Temperatur über einen vorgegebenen Zeitraum
- Entnehmen des Reaktors und Entfernen des Kunststoffstopfens aus der Durchgangsbohrung
- Abfiltern der Planktonprobe durch die untere Filterronde und der Durchgangsbohrung in der Bodenplatte mittels Erzeugen eines Überdruckes am Pumpenanschluss
- ein- oder mehrfaches Wiederholen der letzten sechs Verfahrensschritte mit unterschiedlichen Agenzien
- Trocknen der Planktonprobe mittels Erzeugen eines Überdruckes am Pumpenanschluss
- Demontieren der Bodenplatte mit der unteren Filteranordnung
- Entnehmen der unteren Filterronde und
- Gewinnen des gesäuberten und getrockneten Filtrats mit den mazerierten Mikroplastikpartikeln.

Bevorzugt und vorteilhaft werden dabei als Agenzien Reinstwasser, eine wässrige Lösung mit einem Tensid oder einem Enzym oder Wasserstoffperoxid eingesetzt. Weiterhin werden als Tensid Natriumdodecylsulfat und/oder Enzyme Protease oder Cellulase oder Chitinase oder Lipase bevorzugt und vorteilhaft eingesetzt. Schließlich wird bevorzugt und vorteilhaft das gewonnene Filtrat mit den mazerierten Mikroplastikpartikeln direkt auf der unteren Filterronde einer analytischen Auswertung (beispielsweise ATR-FTIR) zugeführt. Ein kontaminations- und verlustbehaftetes Umfüllen des gereinigten Filtrats entfällt. Nähere Ausführungen zur Anwendung und zur Ausführung des mit der Erfindung beanspruchten Reaktors sind den nachfolgend erläuterten Ausführungsbeispielen zu entnehmen.

### Ausführungsbeispiele

Nachfolgend werden der Reaktor zur Trennung von biogenen und abiogenen Bestandteilen einer Probe durch enzymatische Mazeration nach der Erfindung und seine vorteilhaften Modifikationen sowie das beanspruchte Anwendungsverfahren anhand der schematischen Figuren zum besseren Verständnis der Erfindung an Ausführungsbeispielen noch weitergehend erläutert. Dabei zeigt die
- **Fig.** 1: den schematischen Aufbau des Reaktors nach der Erfindung und
- **Fig.** 2A bis H: den schematischen Verfahrensablauf unter der beanspruchten Anwendung des Reaktors nach der Erfindung.

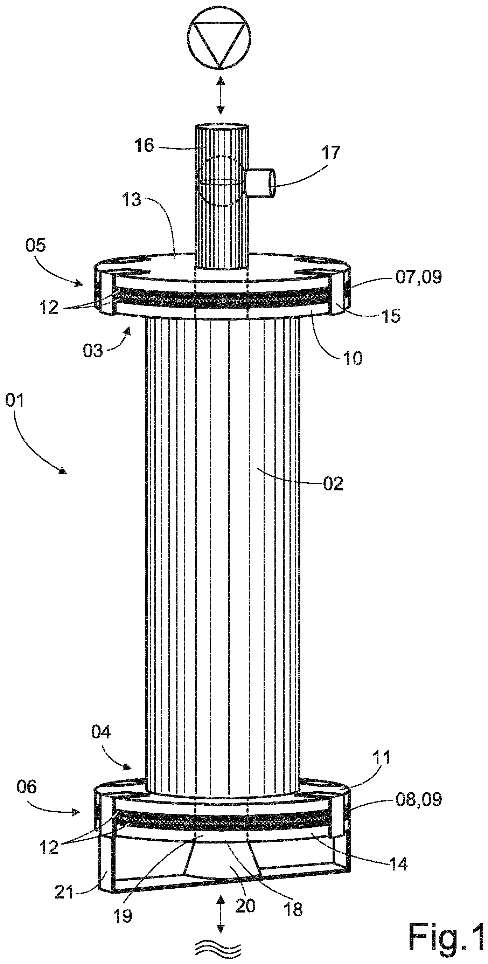

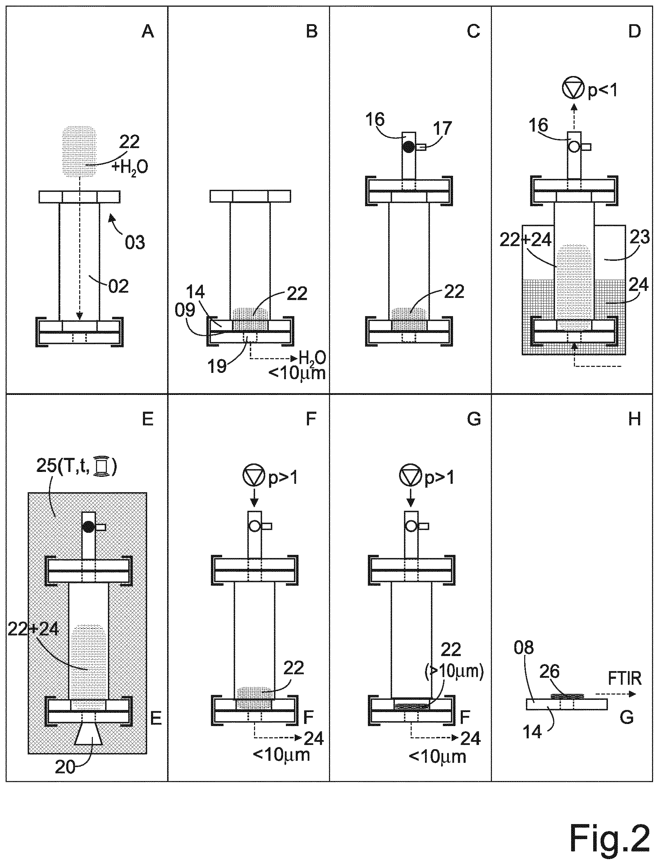

Die **Fig.** 1 zeigt den Reaktor **01** nach der Erfindung in der schematischen Seitenansicht. Zentrales Element des Reaktors **01** ist ein zylindrischer Reaktionsraum **02** mit einer oberen Stirnseite **03** und einer unteren Stirnseite **04.** Im gezeigten Ausführungsbeispiel besteht der Reaktionsraum **02** aus Edelstahl. An der oberen Stirnseite **03** weist der Reaktionsraum **02** eine obere Filteranordnung **05** und an der unteren Stirnseite **04** eine untere Filteranordnung **06** auf. Die obere Filteranordnung **05** weist eine obere Filterronde **07** und die untere Filteranordnung **06** weist eine untere Filterronde **08** auf. Im gezeigten Ausführungsbeispiel handelt es sich jeweils um Edelstahlgaze **09** mit einer Maschenweite von 10 µm. An der oberen Stirnseite **03** weist der Reaktionsraum **02** weiterhin einen oberen Flansch **10** und an der unteren Stirnseite **04** einen unteren Flansch **11** auf. Die Flansche **10, 11** sind in **Fig.** 2 geschnitten dargestellt, sodass der innere Durchgang erkennbar ist. Die obere Filteranordnung **05** ist über zwei Dichtungen **12** (im gezeigten Ausführungsbeispiel Dichtringe aus Silikon) zwischen dem oberen Flansch **10** und einer lösbaren Kopfplatte **13** mittels Spannklammern **15** demontierbar eingespannt. Die untere Filteranordnung **06** ist über zwei weitere Dichtungen **12** zwischen dem unteren Flansch **11** und einer lösbaren Bodenplatte **14** mittels Spannklammern **15** demontierbar eingespannt.

In der Kopfplatte **13** befindet sich ein Pumpenanschluss **16,** der durch ein druckdichtes Ventil **17** verschließbar ist. In der Bodenplatte **14** befindet sich ein Flüssigkeitsanschluss **18.** Im gezeigten Ausführungsbeispiel wird der Flüssigkeitsanschluss **18** von einer einfachen Durchgangsbohrung **19** in der Bodenplatte **14** gebildet. Zum Verschließen des Flüssigkeitsanschlusses **18** wird die Durchgangsbohrung **19** mit einem Kunststoffstopfen **20,** der im dargestellten Ausführungsbeispiel aus Silikon besteht, verschlossen. Damit der Kunststoffstopfen **20** während der Mazeration sicher in der Durchgangsbohrung **19** stecken bleibt, ist er im gewählten Ausführungsbeispiel mittels eines Gestellrahmens **21,** der über die Bodenplatte **14** und den unteren Flansch **11** greift, druckdicht fixiert.

Die Funktionsweise des Reaktors **01** wird anschließend gemäß **Fig.** 2 noch durch die Erläuterung seiner Anwendung in einem Verfahren zur Trennung von biogenen und abiogenen Bestandteilen einer Probe durch enzymatische Mazeration aus einer Mikroplastikpartikel enthaltenden Planktonprobe verdeutlicht. Dabei zeigen die mit Blockbuchstaben bezeichneten einzelnen Bilder verschiedene Verfahrensschritte (hier nicht gezeigte Bezugszeichen sind der **Fig.** 1 zu entnehmen).
**A** Einfüllen einer bezüglich ihres Gehalts an Mikroplastikpartikeln zu untersuchenden wässrigen Planktonprobe **22** + H₂O durch die obere Stirnseite **03** des zylinderförmigen Reaktionsraumes **02.** Bei allen Füll- und Entleerungsvorgängen kann der Reaktor **01** von Hand oder in einem Stativ vertikal gehalten werden (nicht gezeigt).
**B** Abfiltern der Planktonprobe **22** durch die untere Filterronde **08** (in Form einer mit Edelstahlgaze **09** mit einer Maschenweite von 10 µm) und die Durchgangsbohrung **19** in der Bodenplatte **14** mittels Schwerkraft (Filtration). Das Wasser H₂O aus der Planktonprobe **22** und Partikel mit einer Größe < 10 µm verlassen den Reaktionsraum **02**
**C** Montieren der Kopfplatte **13** mit dem Pumpenanschluss **16** und dem druckdichten Ventil **17** an der oberen Stirnseite **03** des Reaktionsraumes **02** unter Zwischenlage der oberen Filteranordnung **05** und der Dichtungen **12** (im Ausführungsbeispiel durch Ansetzen von Spannklammern **15**)
**D** Platzieren (bevorzugt stehend, also vertikal, gehalten von Hand oder in einem Stativ) des Reaktors **01** in einem Gefäß **23** (beispielsweise Becherglas), das mit einem Agens **24** (Reinstwasser, SDS (Natriumdodecylsulfat), Protease, Cellulase, H₂O₂ oder Chitinase,) gefüllt ist. Dabei liegt der Flüssigkeitsanschluss **18** bzw. die Durchgangsbohrung **19** im Agens **24,** also unterhalb dessen Höhenstands im Gefäß **23.** Anschließendes Befüllen des zylinderförmigen Reaktionsraumes **02** mit dem Agens **24** durch Erzeugen eines Vakuums (p<1, also kleiner als 1 bar Luftdruck) am Pumpenanschluss **16.** Das Agens **24** wird aus dem Gefäß **23** in das Innere des Reaktionsraumes **02** gesogen.
**E** Verschließen des druckdichten Ventils **17** (schwarzer Kreis) und der Durchgangsbohrung **19** in der Bodenplatte **14** mit einem Kunststoffstopfen **20** und Positionierung des Reaktors **01** in einem Schüttelinkubator **25.** Die Positionierung des Reaktors **01** erfolgt in der Regel horizontal, also liegend (gezeigt ist eine stehende, also vertikale Positionierung). Der Schüttelinkubator **25** bewegt und erwärmt (beispielsweise über erwärmtes Wasser einer vorgegebenen Temperatur T) die mit dem Agens **24** durchsetzte Planktonprobe **22** stetig über einen längeren Zeitraum t hinweg (siehe Beschreibungseinleitung), sodass die Mazeration, d.h. die Befreiung der anorganischen Partikel von organischen Bestandteilen, erfolgen kann. Nach der Mazeration wird der Reaktor **01** aus dem Schüttelinkubator **25** genommen und der Kunststoffstopfen **20** aus der Durchgangsbohrung **19** entfernt.
**F** Abfiltern der mit dem ersten Agens **24** aufbereiteten Planktonprobe **22** durch die untere Filterronde **08** und der Durchgangsbohrung **19** in der Bodenplatte 14 mittels Erzeugen eines Überdruckes am Pumpenanschluss **16** (Druckfiltration, p>1, also größer als 1 bar Luftdruck). Dazu wird die Pumpe an den Pumpenanschluss **16** angeschlossen und das druckdichte Ventil **17** geöffnet (weißer Kreis).
   **D...F** Anschließend werden die letzten sechs Verfahrensschritte ein- oder mehrfach wiederholt, wobei jedes Mal ein anderes Agens **24** eingesetzt wird. Dadurch wird bei jedem Durchgang ein anderer Mazerationsprozess initiiert, sodass am Ende der Durchläufe die abiogenen Partikel nahezu vollständig von allen biogenen Bestandteilen befreit sind.
**G** vollständiges Entfernen letzter Rückstände der Agenzien **24** und anderer Flüssigkeiten aus der Planktonprobe **22** (Trocknen der Planktonprobe **22**) durch die untere Filterronde **08** und die Durchgangsbohrung **19** in der Bodenplatte **14** mittels Erzeugen eines Überdruckes am Pumpenanschluss **16** (Druckfiltration, p>1, also größer als 1 bar Luftdruck). Ein gesäubertes und getrocknetes Filtrat **26** mit den zu analysierenden Mikroplastikpartikeln (Partikelgröße > 10 µm) bleibt auf der unteren Filterronde **08** zurück.
**H** Demontieren der Bodenplatte **14** mit der unteren Filteranordnung **06** (bzw. umgekehrt Demontieren des Reaktionsraumes **02**) und Entnehmen der unteren Filterronde **08** und Gewinnen des gesäuberten und getrockneten Filtrats **26** mit den aus der Planktonprobe **22** extrahierten Mikroplastikpartikeln.

Das aufbereitete und getrocknete Filtrat **26** wird im gezeigten Ausführungsbeispiel anschließend direkt auf der unteren Filterronde **08** einer analytischen Untersuchung (Partikelauswertung (Anzahl, Größe, Material) beispielsweise durch Fourier transformierte Infrarotanalyse (FTIR) zugeführt.

Als verschiedene Agenzien **24** werden im gezeigten Ausführungsbeispiel Reinstwasser, eine wässrige Lösung mit einem Tensid oder einem Enzym oder Wasserstoffperoxid eingesetzt. Dabei werden als Tensid Natriumdodecylsulfat und/oder Enzyme Protease oder Cellulase oder Chitinase oder Lipase eingesetzt.

### Bezugszeichenliste

- **01**: Reaktor
- **02**: Reaktionsraum
- **03**: obere Stirnseite von 02
- **04**: untere Stirnseite von 02
- **05**: obere Filteranordnung
- **06**: untere Filteranordnung
- **07**: obere Filterronde
- **08**: untere Filterronde
- **09**: Edelstahlgaze
- **10**: oberer Flansch
- **11**: unterer Flansch
- **12**: Dichtung
- **13**: Kopfplatte
- **14**: Bodenplatte
- **15**: Spannklammer
- **16**: Pumpenanschluss
- **17**: druckdichtes Ventil
- **18**: Flüssigkeitsanschluss
- **19**: Durchgangsbohrung
- **20**: Kunststoffstopfen
- **21**: Gestellrahmen
- **22**: Planktonprobe
- **23**: Gefäß
- **24**: Agens
- **25**: Schüttelinkubator
- **26**: gesäubertes und getrocknetes Filtrat

## Patentansprüche

1. Reaktor **(01)** zur enzymatischen Mazeration biogener Bestandteile einer Partikelprobe **(22)** mit zumindest einem Reaktionsraum **(02),** der als vertikaler Zylinder mit einer oberen Stirnseite **(03)** und einer unteren Stirnseite **(04)** ausgebildet ist, zumindest einer Filteranordnung **(05, 06),** einem Pumpenanschluss **(16)** und einem Flüssigkeitsanschluss **(18), dadurch gekennzeichnet, dass** an der oberen Stirnseite **(03)** des Reaktionsraumes **(02)** eine obere Filteranordnung **(05)** mit einer oberen Filterronde **(07)** zwischen zwei Dichtungen **(12)** und an der unteren Stirnseite **(04)** des Reaktionsraumes **(02)** eine untere Filteranordnung **(06)** mit einer unteren Filterronde **(08)** zwischen zwei Dichtungen **(12)** angeordnet ist, und dass auf der oberen Filteranordnung **(05)** eine lösbare Kopfplatte **(13)** mit dem Pumpenanschluss **(16)** und auf der unteren Filteranordnung **(06)** eine lösbare Bodenplatte **(14)** mit dem Flüssigkeitsanschluss **(18)** angeordnet ist, wobei Pumpenanschluss **(16)** und Flüssigkeitsanschluss **(18)** verschließbar und sowohl für eine Befüllung als auch für eine Entleerung des zylinderförmigen Reaktionsraumes **(02)** ausgebildet sind.

2. Reaktor **(01)** nach Anspruch 1, **dadurch gekennzeichnet, dass** die Filterronden **(07, 08)** als Edelstahlgaze **(09)** mit einer Maschenweite von 10 µm ausgebildet sind.

3. Reaktor **(01)** nach Anspruch 1 oder 2, **dadurch gekennzeichnet, dass** die Dichtungen **(12)** als Silikondichtringe ausgebildet sind.

4. Reaktor **(01)** nach einem der vorangehenden Ansprüche, **dadurch gekennzeichnet, dass** an der oberen Stirnseite **(03)** des zylinderförmigen Reaktionsraumes **(02)** ein oberer Flansche **(10)** und an der unteren Stirnseite **(04)** ein unterer Flansch **(11)** angeordnet ist.

5. Reaktor **(01)** nach Anspruch 4, **dadurch gekennzeichnet, dass** die beiden Filteranordnungen **(05, 06)** mittels Spannklammern **(15)** zwischen der Kopfplatte **(13)** und dem oberen Flansch **(10)** und der Bodenplatte **(14)** und dem unteren Flansch **(11)** lösbar eingespannt sind.

6. Reaktor **(01)** nach einem der vorangehenden Ansprüche, **dadurch gekennzeichnet, dass** der Pumpenanschluss **(16)** mit einem druckdichten Ventil **(17)** verschließbar ist.

7. Reaktor **(01)** nach einem der vorangehenden Ansprüche, **dadurch gekennzeichnet, dass** der Flüssigkeitsanschluss **(18)** von einer Durchgangsbohrung **(19)** in der Bodenplatte **(14)** gebildet ist, wobei die Durchgangsbohrung (19) mittels eines Kunststoffstopfens **(20)** druckdicht verschließbar ist.

8. Reaktor **(01)** nach Anspruch 7, **dadurch gekennzeichnet, dass** der Kunststoffstopfen **(20)** mittels eines Gestellrahmens **(21)** druckdicht in der Durchgangsbohrung **(19)** halterbar ist.

9. Reaktor **(01)** nach Anspruch 7 oder 8, **dadurch gekennzeichnet, dass** der Kunststoffstopfen **(20)** aus Silikon besteht.

10. Verwendung des Reaktors **(01)** nach einem der vorangehenden Ansprüche in einem Verfahren zur enzymatischen Mazeration biogener Bestandteile einer Mikroplastikpartikel enthaltenden Planktonprobe **(22)** mit den Verfahrensschritten:
• Einfüllen der Planktonprobe **(22)** durch die obere Stirnseite **(03)** des zylinderförmigen Reaktionsraumes **(02)**
• Abfiltern der Planktonprobe **(22)** durch die untere Filterronde **(08)** und der Durchgangsbohrung **(19)** in der Bodenplatte **(14)** mittels Schwerkraft
• Montieren der Kopfplatte **(13)** mit dem Pumpenanschluss **(16)** und dem druckdichten Ventil **(17)** unter Zwischenlage der oberen Filteranordnung **(05)** und der Dichtungen **(12)**
• Platzieren des Reaktors **(01)** in einem Gefäß **(23),** das mit einem Agens **(24)** gefüllt ist,
• Befüllen des zylinderförmigen Reaktionsraumes **(02)** mit dem Agens **(24)** durch Erzeugen eines Vakuums am Pumpenanschluss **(16)**
• Verschließen des Ventils **(17)** im Pumpenanschluss **(16)** und der Durchgangsbohrung **(19)** in der Bodenplatte **(14)** mit einem Kunststoffstopfen **(20)** und Positionierung des Reaktors **(01)** in einem Schüttelinkubator **(25)**
• Inkubieren des Reaktors **(25)** bei einer vorgegebenen Temperatur T über einen vorgegebenen Zeitraum t
• Entnehmen des Reaktors **(01)** und Entfernen des Kunststoffstopfens **(20)** aus der Durchgangsbohrung **(19)**
• Abfiltern der Planktonprobe **(22)** durch die untere Filterronde **(08)** und die Durchgangsbohrung **(19)** in der Bodenplatte **(14)** mittels Erzeugen eines Überdruckes am Pumpenanschluss **(16)**
• ein- oder mehrfaches Wiederholen der letzten sechs Verfahrensschritte mit unterschiedlichen Agenzien **(24)**
• Trocknen der Planktonprobe **(22)** mittels Erzeugen eines Überdruckes am Pumpenanschluss **(16)**
• Demontieren der Bodenplatte **(14)** mit der unteren Filteranordnung **(06)**
• Entnehmen der unteren Filterronde **(08)** und
• Gewinnen des gesäuberten und getrockneten Filtrats **(26)** mit den mazerierten Mikroplastikpartikeln.

11. Verwendung des Reaktors **(01)** nach Anspruch 10 **dadurch gekennzeichnet, dass** als Agenzien **(24)** Reinstwasser, eine wässrige Lösung mit einem Tensid oder einem Enzym oder Wasserstoffperoxid eingesetzt werden.

12. Verwendung des Reaktors **(01)** nach Anspruch 11 **dadurch gekennzeichnet, dass** als Tensid Natriumdodecylsulfat und/oder Enzyme Protease oder Cellulase oder Chitinase oder Lipase eingesetzt werden.

13. Verwendung des Reaktors **(01)** nach einem der vorangehenden Ansprüche, **dadurch gekennzeichnet, dass** das gewonnene, gesäuberte und getrocknete Filtrat **(26)** mit den mazerierten Mikroplastikpartikeln direkt auf der unteren Filterronde **(08)** einer analytischen Auswertung zugeführt wird.

## Claims

1. A reactor (01) for the enzymatic maceration of biogenic constituents of a particle sample (22) with at least one reaction chamber (02), which is configured as a vertical cylinder with an upper face side (03) and a lower face side (04), at least one filter arrangement (05, 06), a pump connection (16) and a fluid connection (18), **characterized in that** at the upper face side (03) of the reaction chamber (02) an upper filter arrangement (05) is arranged with an upper filter disc (07) between two seals (12), and on the lower face side (04) of the reaction chamber (02) a lower filter arrangement (06) is arranged with a lower filter disc (08) between two seals (12), and that on the upper filter arrangement (05) a detachable head plate (13) with the pump connection (16) is arranged, and on the lower filter arrangement (06) a detachable base plate (14) with the fluid connection (18) is arranged, wherein pump connection (16) and fluid connection (18) are able to be closed and are configured both for a filling and also for an emptying of the cylindrical reaction chamber (02).

2. The reactor (01) according to claim 1, **characterized in that** the filter discs (07, 08) are configured as high-grade steel gauze (09) with a mesh width of 10 µm.

3. The reactor (01) according to claim 1 or 2, **characterized in that** the seals (12) are configured as silicone sealing rings.

4. The reactor (01) according to one of the preceding claims, **characterized in that** on the upper face side (03) of the cylindrical reaction chamber (02) an upper flange (10) is arranged, and on the lower face side (04) a lower flange (11) is arranged.

5. The reactor (01) according to claim 4, **characterized in that** the two filter arrangements (05, 06) are clamped detachably by means of tension clamps (15) between the head plate (13) and the upper flange (10) and the base plate (14) and the lower flange (11).

6. The reactor (01) according to one of the preceding claims, **characterized in that** the pump connection (16) is able to be closed by a pressure-tight valve (17).

7. The reactor (01) according to one of the preceding claims, **characterized in that** the fluid connection (18) is formed by a through-bore (19) in the base plate (14), wherein the through-bore (19) is able to be closed in a pressure-tight manner by means of a plastic plug (20).

8. The reactor (01) according to claim 7, **characterized in that** the plastic plug (20) is able to be mounted in the through-bore (19) in a pressure-tight manner by means of a rack frame (21) .

9. The reactor (01) according to claim 7 or 8, **characterized in that** the plastic plug (20) consists of silicone.

10. A use of the reactor (01) according to one of the preceding claims in a method for the enzymatic maceration of biogenic constituents of a plankton sample (22) containing microplastic particles, with the method steps:
• filling the plankton sample (22) in through the upper face side (03) of the cylindrical reaction chamber (02)
• filtering off the plankton sample (22) through the lower filter disc (08) and the through-bore (19) in the base plate (14) by means of gravity
• mounting the head plate (13) with the pump connection (16) and the pressure-tight valve (17) with interposing of the upper filter arrangement (05) and of the seals (12)
• placing of the reactor (01) in a vessel (23) which is filled with an agent (24)
• filling the cylindrical reaction chamber (02) with the agent (24) by generating a vacuum at the pump connection (16)
• closing the valve (17) in the pump connection (16) and the through-bore (19) in the base plate (14) with a plastic plug (20) and positioning of the reactor (01) in an incubator shaker (25)
• incubating the reactor (25) at a predetermined temperature T over a predetermined time period t
• withdrawing the reactor (01) and removing the plastic plug (20) from the through-bore (19)
• filtering off the plankton sample (22) through the lower filter disc (08) and the through-bore (19) in the base plate (14) by means of generating an excess pressure at the pump connection (16)
• single or multiple repetition of the last six method steps with different agents (24)
• drying the plankton sample (22) by means of generation of an excess pressure at the pump connection (16)
• disassembling the base plate (14) with the lower filter arrangement (06)
• removing the lower filter disc (08) and
• obtaining the cleaned and dried filtrate (26) with the macerated microplastic particles.

11. The use of the reactor (01) according to claim 10, **characterized in that** as agents (24) ultrapure water, an aqueous solution with a tenside or with an enzyme or hydrogen peroxide are used.

12. The use of the reactor (01) according to claim 11, **characterized in that** as tenside, sodium dodecylsulphate and/or enzymes protease or cellulase or chitinase or lipase are used.

13. The use of the reactor (01) according to one of the preceding claims, **characterized in that** the obtained, cleaned and dried filtrate (26) with the macerated microplastic particles is delivered directly on the lower filter disc (08) to an analytical evaluation.

## Revendications

1. Réacteur (01) pour la macération enzymatique de constituants biogéniques d'un échantillon de particules (22), doté d'au moins une chambre réactionnelle (02), qui est conçue sous la forme d'un cylindre vertical avec une face frontale (03) supérieure et une face frontale (04) inférieure, d'au moins un agencement de filtrage (05, 06), d'un raccord sur pompe (16) et d'un branchement de liquide (18), **caractérisé en ce que** sur la face frontale (03) supérieure de la chambre réactionnelle (02) est placé un agencement de filtrage (05) supérieur, pourvu d'un disque filtrant (07) supérieur entre deux joints (12) et sur la face frontale (04) inférieure de la chambre réactionnelle (02) est placé un agencement de filtrage (06) inférieur, pourvu d'un disque filtrant (08) inférieur entre deux joints (12) et **en ce que** sur l'agencement de filtrage (05) supérieur est placée une plaque de tête (13) amovible avec le raccord sur pompe (16) et sur l'agencement de filtrage (06) inférieur est placée une plaque de base (14) amovible avec le branchement de liquide (18), le raccord sur pompe (16) et le branchement de liquide (18) pouvant se fermer et étant conçus aussi bien pour un remplissage qu'également pour un vidage de la chambre réactionnelle (02) de forme cylindrique.

2. Réacteur (01) selon la revendication 1, **caractérisé en ce que** les disques filtrants (07, 08) sont conçus en tant que toile en acier inoxydable (09) d'un maillage de 10 µm.

3. Réacteur (01) selon la revendication 1 ou 2, **caractérisé en ce que** les joints (12) sont conçus en tant que bagues d'étanchéité en silicone.

4. Réacteur (01) selon l'une quelconque des revendications précédentes, **caractérisé en ce que** sur la face frontale (03) supérieure de la chambre réactionnelle (02) de forme cylindrique est placée une bride (10) supérieure et sur la face frontale (04) inférieure est placée une bride (11) inférieure.

5. Réacteur (01) selon la revendication 4, **caractérisé en ce que** les deux agencements de filtrage (05, 06) son enserrés de manière amovible au moyen de crampons de serrage (15) entre la plaque de tête (13) et la bride (10) supérieure et entre la plaque de base (14) et la bride (11) inférieure.

6. Réacteur (01) selon l'une quelconque des revendications précédentes, **caractérisé en ce que** le raccord sur pompe (16) peut se fermer à l'aide d'une soupape (17) étanche à la pression.

7. Réacteur (01) selon l'une quelconque des revendications précédentes, **caractérisé en ce que** le branchement de liquide (18) est formé par un perçage traversant (19) dans la plaque de base (14), le perçage traversant (19) pouvant se fermer de manière étanche à la pression par un bouchon en matière plastique (20).

8. Réacteur (01) selon la revendication 7, **caractérisé en ce que** le bouchon en matière plastique (20) est susceptible d'être maintenu de manière étanche à la pression dans le perçage traversant (19) au moyen d'un cadre d'armature (21) .

9. Réacteur (01) selon la revendication 7 ou 8, **caractérisé en ce que** le bouchon en matière plastique (20) consiste dans du silicone.

10. Utilisation du réacteur (01) selon l'une quelconque des revendications précédentes dans un procédé de macération enzymatique de constituants biogéniques d'un échantillon de plancton (22) contenant des microparticules de plastique, comportant les étapes consistant à :
• verser l'échantillon de plancton (22) à travers la face frontale (03) supérieure de la chambre réactionnelle (02) de forme cylindrique,
• filtrer l'échantillon de plancton (22) à travers le disque filtrant (08) inférieur et le perçage traversant (19) dans la plaque de base (14) au moyen de la gravité,
• monter la plaque de tête (13) avec le raccord sur pompe (16) et la soupape (17) étanche à la pression, en intercalant l'agencement de filtrage supérieur (05) et les joints (12),
• placer le réacteur (01) dans un contenant (23), qui est rempli d'un agent (24),
• remplir la chambre réactionnelle (02) de forme cylindrique avec l'agent (24), par génération d'un vide sur le raccord sur pompe (16),
• fermer la soupape (17) dans le raccord sur pompe (16) et dans le perçage traversant (19) dans la plaque de base (14) avec un bouchon en matière plastique (20) et positionner le réacteur (01) dans un incubateur vibrant (25),
• faire incuber le réacteur (25) à une température prédéfinie T sur une période prédéfinie t,
• retirer le réacteur (01) et enlever le bouchon en matière plastique (20) du perçage traversant (19),
• filtrer l'échantillon de plancton (22) à travers le disque filtrant (08) inférieur et le perçage traversant (19) dans la plaque de base (14) par création d'une surpression sur le raccord sur pompe (16),
• répéter une ou plusieurs fois les six dernières étapes de procédé avec différents agents (24)
• faire sécher l'échantillon de plancton (22) par création d'une surpression sur le raccord sur pompe (16)
• démonter la plaque de base (14) avec l'agencement de filtrage (06) inférieur,
• retirer le disque filtrant (08) inférieur et
• récupérer le produit filtré (26) purifié, avec les microparticules de plastique macérées.

11. Utilisation du réacteur (01) selon la revendication 10, **caractérisée en ce qu'**en tant qu'agents (24), on met en oeuvre de l'eau extrêmement pure, une solution aqueuse avec un tensioactif ou un enzyme ou du peroxyde d'hydrogène.

12. Utilisation du réacteur (01) selon la revendication 11, **caractérisé en ce qu'**en tant que tensioactif, on met en oeuvre du dodécylsulfate de sodium et/ou des enzymes protéases ou cellulases ou chitinases ou lipases.

13. Utilisation du réacteur (01) selon l'une quelconque des revendications précédentes, **caractérisé en ce qu'**on amène le produit filtré (26) purifié séché récupéré avec les microparticules macérées de plastique directement sur le disque filtrant (08) inférieur d'une évaluation analytique.
